# EUROPÄISCHE PATENTSCHRIFT

(11) **EP 1 666 074 B1**
(45) Veröffentlichungstag und Bekanntmachung des Hinweises auf die Patenterteilung: **28.05.2008**
(21) Anmeldenummer: 04028174.3
(22) Anmeldetag: 26.11.2004
(51) Int. Cl.: A61L 2/10, A61L 9/20, F24F 3/16

(54) **Entkeimungsleuchte**
Disinfection lamp
Lampe de désinfection

(43) Veröffentlichungstag der Anmeldung: 07.06.2006
(73) Patentinhaber: BÄ*RO GmbH & Co. KG, 42799 Leichlingen (DE)
(72) Erfinder: Ferres, Martin, 42655 Soligen (DE); Kirsten, Martin Dr., 51399 Burscheid (DE); Becker, Siegfried, 42655 Solingen (DE)
(74) Vertreter: Paul, Dieter-Alfred

(56) Entgegenhaltungen:
- WO-A-02/089859
- DE-A- 19 653 083
- DE-C- 972 572
- US-A- 4 048 537
- US-A1- 2003 214 256

## Beschreibung

Die vorliegende Erfindung betrifft eine UV-C-Entkeimungsleuchte gemäß der Präambel des Anspruch 1.

Eine solche Entkeimungsleuchte ist aus der DE-196 53 083 A1 bekannt.

Zur Entkeimung von Luft in gewerblich und industriell genutzten Räumen sowie in Krankenhäusern oder Flugzeugen werden UV-C-Entkeimungsleuchten eingesetzt. Ihre keimtötende UV-C-Linienstrahlung mit einer Wellenlänge von = 253,75 nm wird verwendet, um Bakterien, Pilze oder Viren oder andere Mikroorganismen abzutöten.

Ein weiteres Einsatzgebiet stellt die Lebensmittelproduktion dar, in welcher die UV-C-Entkeimungsleuchten zur Entkeimung von Verpackungsmaterialien für Lebensmittel, von Betriebsmitteln und zur Luftentkeimung eingesetzt werden.

In der US 4,048,537 ist eine UV-C-Entkeimungsleuchte mit einem langgestreckten Strahlergrundkörper und einem elektrischen Anschluß und einem Sockel offenbart, bei der der Strahlergrundkörpermit einer Schutzhülle aus Teflon umgeben ist.

Eine weitere UV-C-Entkeimungsleuchte ist in der WO 02/089859 A beschrieben. Sie weist ebenfalls einen langgestreckten Strahlergrundkörper mit einem elektrischen Anschluß sowie eine für UV-C-Strahlung durchlässige flexible Schutzhülle auf.

Nachteilig an den bekannten UV-C-Entkeimungsleuchten ist, daß der Strahler aus dem Sockel heraus Fallen kann, wenn beispielsweise bei dessen Demontage das Vergußmaterial bereits aus dem Sockel entfernt wurde.

Der Erfindung liegt daher die Aufgabe zugrunde, eine Verbesserung der bekannten UV-C-Entkeimungsleuchten bereitzustellen, die die oben genannten Probleme löst.

Diese Aufgabe wird durch eine UV-C-Entkeimungsleuchte mit den Merkmalen des kennzeichenden Teils des Anspruchs 1 gelöst.

Gemäß einer ersten Ausführungsform der Erfindung kann die Schutzhülle aus Teflon bestehen. Deren Dicke kann je nach Anwendung zwischen 0,1 mm und 0,5 mm liegen, wobei sie vorzugsweise 0,3 mm betragen kann.

Gemäß einer weiteren Ausführung der Erfindung kann das Vergußmaterial UV-C-strahlungsbeständig sein.

Es ist zweckmäßig, den Sockel aus einem Keramikgehäuse zu bilden, wobei es kostengünstig ist, letzteres insbesondere mit Glaswolle als Füllmaterial zu füllen.

Die Betriebstechnik für den Strahler kann separat außerhalb der UV-C-Entkeimungsleuchte angeordnet und über eine Verbindung der externen Betriebstechnik mit an dem Sockel der UV-C-Entkeimungsleuchte angeordneten Kontakten den UV-C-Strahler mit Strom versorgt werden.

Alternativ kann der UV-C-Strahler auch als High-Output-Strahler oder als herkömmlicher UV-C-Kompaktniederdruckstrahler mit zwei Entladungsrohren ausgeführt sein.

Hinsichtlich weiterer vorteilhafter Ausgestaltungen und Weiterbildungen der Erfindung wird auf die Unteransprüche sowie die nachfolgende Beschreibung eines Ausführungsbeispiels anhand der beiliegenden Zeichnung verwiesen. In der Zeichnung zeigt:
- Figur 1: eine perspektivische Darstellung einer erfindungsgemäßen UV-C-Entkeimungsleuchte; und
- Figur 2: die UV-C-Entkeimungsleuchte gemäß Figur 1 von unten.

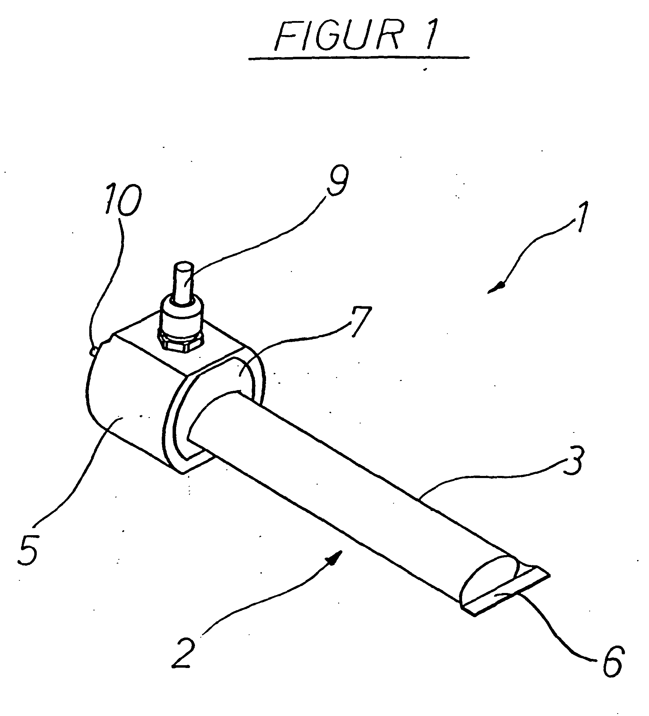

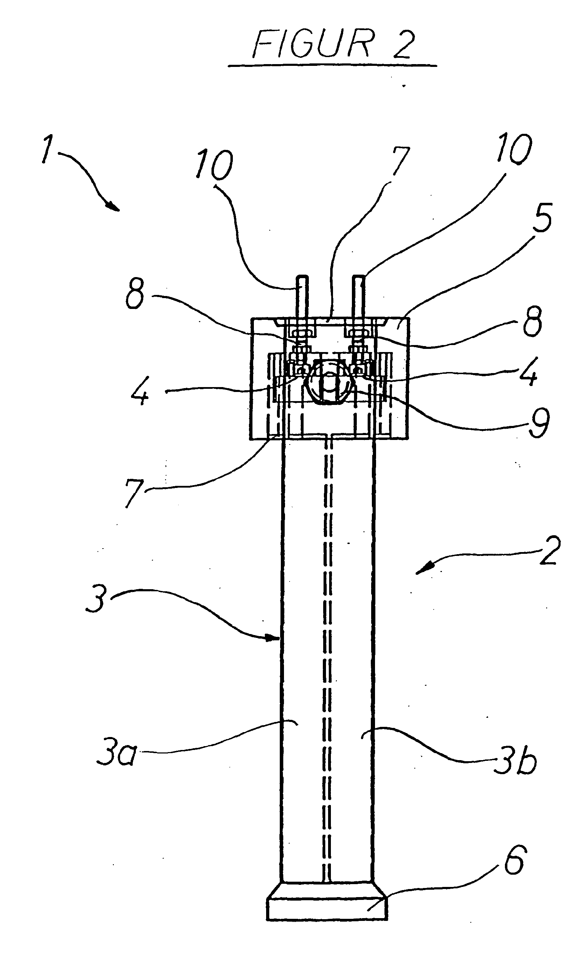

Die Figuren 1 und 2 zeigen ein Ausführungsbeispiel einer erfindungsgemäßen UV-C-Entkeimungsleuchte 1.

Die UV-C-Entkeimungsleuchte 1 weist einen UV-C-Strahler 2 in Form eines High-Output-UV-C-Kompaktniederdruckstrahlers auf. Der UV-C-Strahler 2 besitzt einen Strahlergrundkörper 3, hier beispielhaft mit zwei Entladungsrohren 3a, 3b gezeigt, und elektrische Anschlüsse 4, über die der UV-C-Strahler 2 mit einem Sockel 5 verbunden ist. Der Strahlergrundkörper 3 ist vollständig von einer für UV-C-Strahlung durchlässigen flexiblen Schutzhülle 6 aus Teflon mit einer Foliendicke von ungefähr 0,3 mm umgeben.

Im Bereich seiner elektrischen Anschlüsse 4 ist der UV-C-Strahler 2 über eine Klemme (nicht dargestellt) in dem Sockel gesichert und mittels eines UV-C-strahlungsbeständigen Vergußmaterials 7 in den Sockel 5 eingegossen, um die in dem Sockel 5 angeordnete Elektrik gegen ein Eindringen von Feuchtigkeit zu schützen. In diesem Ausführungsbeispiel sind der Sockel 5 und der UV-C-Strahler 2 zusätzlich über Schraubenverbindungen 8 miteinander verbunden. Die Schraubenverbindungen 8 sind ebenfalls, beispielsweise durch das Vergußmaterial, gegenüber der Umgebung abgedichtet. An der Oberseite des Sockels 5 ist eine Kabelzuführung 9 vorgesehen.

An der dem UV-C-Strahler abgewandten Seite weist der Sokkel 5 Kontakte 10 auf, über die die UV-C-Entkeimungsleuchte zur Stromversorgung mit außerhalb der UV-C-Entkeimungsleuchte 1 angeordneter Betriebstechnik (nicht dargestellt) verbunden werden kann.

Die Schutzhülle 6 ist an der dem Sockel abgewandten Ende des UV-C-Strahlers 2 beispielsweise verschweißt, so daß der in der Schutzhülle 6 befindliche UV-C-Strahler 2 vollständig von der Schutzhülle 6 umschlossen ist. Dadurch wird er gegenüber der Umgebung abgedichtet und somit vor Feuchtigkeit, insbesondere vor Spritz- bzw. Strahlwasser geschützt. Bei einem eventuellen Bruch des Strahlergrundkörpers 3 können somit keine Splitter unbemerkt auf ein unter der UV-C-Entkeimungsleuchte 1 angeordnete Behandlungsgut oder in den Kreislauf von Entkeimungsanlagen wie beispielsweise Luftentkeimungsanlagen gelangen und diese verunreinigen, sondern verbleiben innerhalb der Schutzhülle 6. Die Schutzhülle 6 hat zusätzlich die Funktion als Wärmeisolationsschutz, so daß eine Abnahme der Strahlungsleistung im Einsatz in kalten Umgebungen verhindert wird.

## Patentansprüche

1. UV-C-Entkeimungsleuchte (1) zur Behandlung eines Mediums mit UV-C-Strahlung, um die darin enthaltenen Mikroorganismen abzutöten, mit mindestens einem UV-C-Strahler (2), der einen langgestreckten Strahlergrundkörper (3) aufweist, welcher von einer für UV-C-Strahlung durchlässigen flexiblen Schutzhülle (6) umgeben ist, mindestens einem elektrischen Anschluß (4), und einem den UV-C-Strahler (2) aufnehmenden Sockel (5), wobei der UV-C-Strahler (2) im Bereich seiner elektrischen Anschlüsse (4) mittels eines Vergußmaterials (7) in den Sockel (5) eingegossen ist, **gekennzeichnet durch** eine im Sockel (5) angeordnete Klemme, **durch** die der UV-C-Strahler (2) gegen ein ungewolltes Herausnehmen bzw. Herausfallen aus dem Sockel (5) gesichert ist, wenn das vergußmaterial (7) bereits aus dem Sockel (5) entfernt wurde.

2. UV-C-Entkeimungsleuchte (1) nach Anspruch 1, **dadurch gekennzeichnet, daß** die Schutzhülle (6) aus Teflon ist.

3. UV-C-Entkeimungsleuchte nach Anspruch 1 oder 2, **dadurch gekennzeichnet, daß** die Schutzhülle (6) eine Dicke zwischen 0,1 mm und 0,5 mm, vorzugsweise von 0,3 mm, aufweist.

4. UV-C-Entkeimungsleuchte (1) nach einem der vorherigen Ansprüche, **dadurch gekennzeichnet, daß** das Vergußmaterial (7) UV-C-strahlungsbeständig ist.

5. UV-C-Entkeimungsleuchte (1) nach einem der vorhergehenden Ansprüche, **dadurch gekennzeichnet, daß** der Sockel (5) aus einem Keramikgehäuse gebildet ist, das insbesondere mit Glaswolle als Füllmaterial gefüllt ist.

6. UV-C-Entkeimungsleuchte (1) nach einem der vorhergehenden Ansprüche, **dadurch gekennzeichnet, daß** eine Betriebstechnik für den UV-C-Strahler (2) separat außerhalb der UV-C-Entkeimungsleuchte (1) angeordnet ist.

7. UV-C-Entkeimungsleuchte (1) nach einem der vorhergehenden Ansprüche, **dadurch gekennzeichnet, daß** der UV-C-Strahler (2) ein Kompaktstrahler ist.

8. UV-C-Entkeimungsleuchte (1) nach einem der Ansprüche 1 bis 6, **dadurch gekennzeichnet, daß** der UV-C-Strahler (2) ein High-Output-Strahler ist.

9. UV-C-Entkeimungsleuchte (1) nach einem der Ansprüche 1 bis 5, **dadurch gekennzeichnet, daß** der UV-C-Strahler (1) ein High-Output-UV-C-Kompaktniederdruckstrahler mit zwei Entladungsrohren (3a, 3b) ist.

## Claims

1. UV-C sterilizing lamp (1) for the treatment of a medium with UV-C radi a-tion, in order to kill the microorganisms contained therein, with at least one UV-C emitter (2), which has an elongated emitter foundation (3) that is su r-rounded by a flexible protective cover (6), which is radiolucent to UV-C radiation, at least one electrical connection (4), and a base (5) containing the UV-C emitter (2), that is cast into the base (5) in the area of its electrical connections (4) by means of casting material (7), **characterized by** a clamp arranged in the base (5) by which the UV-C emitter (2) is secured against involuntary removal or falling out of the base (5), when the casting material (7) has been removed from the base (5).

2. UV-C sterilizing lamp (1) according to claim 1 **characterized in that** the protective cover (6) is made of Teflon.

3. UV-C sterilizing lamp (1) according to claim 1 or 2, **characterized in that** the protective cover has a thickness of 0.1 mm to 0.5 mm, preferably of 0.3 mm.

4. UV-C sterilizing lamp (1) according to any preceding claim, **characterized in that** the casting material (7) is resistant to UV-C radiation.

5. UV-C sterilizing lamp (1) according to one of the preceding claims **characterized in that** the base (5) is formed from a ceramics body, which is especially filled with glass wool as filling material.

6. UV-C sterilizing lamp (1) according to one of the preceding claims **characterized in that** an operating technology for the UV-C emitter (2) is arranged separately outside of the UV-C sterilizing lamp (1).

7. UV-C sterilizing lamp (1) according to one of the preceding claims **characterized in that** the UV-C emitter (2) is a compact emitter.

8. UV-C sterilizing lamp (1) according to one of the claims 1 to 6 **characterized in that** the UV-C emitter (2) is a high output emitter.

9. UV-C sterilizing lamp (1) according to one of the claims 1 to 6 **characterized in that** the UV-C emitter (1) is a high output UV-C compact low pressure emitter with two discharge tubes (3a, 3b).

## Revendications

1. Lampe de désinfection UV-C (1) pour traitement d'un médium avec des rayonnements UV-C, dans le but de tuer les microorganismes existants dans ce dernier, comportant, au moins une lampe UV-C (2), qui présente un corps de base allongé (3) entouré d'une housse de protection (6) flexible et perméable aux rayons UV-C, au moins un raccordement électrique (4), et un socle (5) incorporant la lampe UV-C (2), de telle façon que la lampe UV-C (2) est coulée aux niveaux de ces raccordements électriques (4), dans le socle (5), à l'aide d'un matériel de scellement (7), **caractérisée en ce que** la mise en oeuvre d'une pince au niveau du socle (5), assure contre un prélèvement involontaire resp. une tombée de la lampe UV-C, si le matériel de scellement (7) a été enlevé du socle (5).

2. Lampe de désinfection UV-C (1) selon la revendication 1, **caractérisée en ce que** la housse protectrice (6) est constituée de téflon.

3. Lampe de désinfection UV-C (1) selon la revendication 1 ou 2, **caractérisée en ce que** la housse protectrice (6) présente une épaisseur entre 0,1 et 0,5 mm, de préférence une épaisseur de 0,3 mm.

4. Lampe de désinfection UV-C (1) selon l'une des revendications préalables, **caractérisée en ce que** le matériel de scellement (7) est résistant aux rayonnements UV-C.

5. Lampe de désinfection UV-C (1) selon l'une des revendications préalables, **caractérisée en ce que** le socle est formé d'un boîtier en céramique, rempli en particulier de laine de verre comme matériau de remplissage.

6. Lampe de désinfection UV-C (1) selon l'une des revendications précédentes, **caractérisée en ce que** un dispositif technique pour le fonctionnement de la lampe UV-C (2), est disposé séparément, à l'extérieur de la lampe de désinfection UV-C (1).

7. Lampe de désinfection UV-C (1) selon l'une des revendications précédentes, **caractérisée en ce que** la lampe UV-C (2) présente une unité compacte.

8. Lampe de désinfection UV-C (1) selon l'une des revendications 1 à 6, **caractérisée en ce que** la lampe UV-C (2) est une lampe à haute flux sortants.

9. Lampe de désinfection UV-C (1) selon l'une des revendications 1 à 5, **caractérisée en ce que** la lampe UV-C (2) est une lampe UV-C compacte à hautes flux sortants et basse pression avec deux tuyaux de décharge (3a, 3b).
